# FASCICULE DE BREVET EUROPEEN

(11) **EP 3 427 740 B1**
(45) Date de publication et mention de la délivrance du brevet: **26.01.2022**
(21) Numéro de dépôt: 18305865.0
(22) Date de dépôt: 02.07.2018
(51) Int. Cl.: A61K 31/6615, A61K 33/14, A61P 35/00, A61K 31/047

(54) **NOUVEAU TRAITEMENT DU CANCER PAR UNE COMBINAISON D'UN DÉRIVÉ PHOSPHORIQUE D'INOSITOL ET DE CHLORURE DE MAGNÉSIUM**
NEUE KREBSTHERAPIE MIT HILFE EINER KOMBINATION AUS EINEM INOSITOLPHOSPHOR DERIVAT UND MAGNESIUMCHLORID
NOVEL CANCER TREATMENT WITH A COMBINATION OF INOSITOL PHOSPHATE DERIVATIVE AND MAGNESIUM CHLORIDE

(30) Priorité: 10.07.2017 FR 1756494
(43) Date de publication de la demande: 16.01.2019
(73) Titulaire: Parc, Guy, 92500 Rueil Malmaison (FR)
(72) Inventeur: Parc, Guy, 92500 Rueil Malmaison (FR)
(74) Mandataire: Jacobacci Coralis Harle

(56) Documents cités:
- EP-A1- 2 452 577
- US-A- 4 952 396
- KHATIWADA J ET AL: "Combination of green tea, phytic acid, and inositol reduced the incidence of azoxymethane-induced colon tumors in Fisher 344 male rats", LWT- FOOD SCIENCE AND TECHNOLOGY, ACADEMIC PRESS, UNITED KINGDOM, vol. 39, no. 10, 1 décembre 2006 (2006-12-01), pages 1080-1086, XP024953805, ISSN: 0023-6438, DOI: 10.1016/J.LWT.2005.07.018 [extrait le 2006-12-01]
- R. H. CRAIG: "The value of magnesium chloride as an aid in the treatment of cancer", CANADIAN MEDICAL ASSOCIATION JOURNAL, vol. 31, no. 5, novembre 1934 (1934-11), pages 531-533, XP002781310,
- T. MAFFUCCI ET AL: "Inhibition of the phosphatidylinositol 3-kinase/Akt pathway by inositol pentakisphosphate results in antiangiogenic and antitumor effects", CANCER RESEARCH, vol. 65, no. 18, 15 septembre 2005 (2005-09-15), pages 8339-8349, XP002781309,

## Description

La présente invention se rapporte au domaine du traitement thérapeutique des cancers chez l'homme.

L'invention concerne en particulier l'utilisation en médecine humaine d'une composition pour son activité anticancéreuse.

Selon des statistiques récentes de l'organisation mondiale de la santé (OMS), le cancer représente la deuxième cause de mortalité après les maladies cardiovasculaires dans les pays industrialisés. Les moyens mis en oeuvre pour faire face aux maladies cancéreuses passent par le renforcement du diagnostic précoce, mais aussi par l'amélioration des traitements médicamenteux. La découverte de nouvelles molécules ou compositions originales, dont la spécificité à l'égard des cellules tumorales serait totale par rapport aux cellules saines, permettrait la mise au point de nouvelles thérapies.

Avant d'effectuer un choix thérapeutique pour le traitement d'un cancer, un certain nombre de paramètres doivent être étudiés : le type de cancer (sarcomes, mélanomes...), l'organe atteint, le stade d'évolution du cancer, l'ensemble des facteurs pronostics et les caractéristiques propres au malade (âge, état général, état psychique...). A partir de ces données, une thérapie peut être choisie, soit locale, soit générale. Les traitements les plus efficaces sont des thérapies locales mettant en jeu des méthodes de chirurgie et/ou de radiothérapie. Elles traitent les lésions peu développées et guérissent la plupart des cancers localisés. Les thérapies générales, chimiothérapie et/ou l'hormonothérapie, sont en général des traitements palliatifs ou adjuvants. Ces traitements sont mis en oeuvre dans le cas de cancers localisés mais plus développés. Ils permettent de guérir un nombre limité de cancers généralisés mais améliorent l'espérance de vie des malades (cf. Capdeville R., Buchdunger E., Zimmermann J., Matter A., Nature Rev. Drug Discov., 2002, 1, 493; Eisenberg B. L., Von Mehren M., Expert Opin Pharmacother., 2003, 6, 869; Gilman A., Philips F. S., Science, 1946, 103, 409; Gingras D, Béliveau R. Med. Sci., 1997, 13, 1428-35.

La limitation concernant l'utilisation des anticancéreux connus concerne leur forte toxicité, qui est à l'origine d'un grand nombre d'effets secondaires pouvant aller jusqu'à la mort du patient. Les armes chimiques utilisées pour le traitement du cancer sont censées détruire les cellules cancéreuses en épargnant les cellules saines. Mais la sélectivité est toute relative, et la plupart des médicaments utilisés en chimiothérapie présentent une toxicité hématologique non négligeable. Réduire les effets secondaires néfastes, surtout ceux aux conséquences graves sur le plan médical et psychologique, est aussi important que de tenter d'améliorer l'efficacité d'un médicament donné. Dans l'ensemble, l'arsenal actuel du chimiothérapeute se compose encore de médicaments forts cytotoxiques, anciens, la plupart des anticancéreux connus ayant déjà plusieurs dizaines d'années, bien peu ciblés, sur le plan cellulaire tout au moins, et n'offrant aucune alternative aux phénomènes de résistance. Il existe donc un besoin en nouvelles compositions anticancéreuses, utilisables en chimiothérapie, qui, idéalement, ne cibleraient que les cellules cancéreuses.

C'est lors de ses longs séjours en Afrique, dans les années 1970, que le Docteur Burkitt avait remarqué une corrélation entre une alimentation particulièrement riche en céréales complètes et donc en fibres et la faible incidence du cancer colorectal, du sein, de la prostate et de l'endomètre comparativement à celle des européens (Burkitt D. P., Dietary fiber and Cancer, J. Nut. 1988 118: 531-533). Dans son actualisation parue en 2015 (« Nutrition et Prévention Primaire des Cancers; Actualisation des Données juin 2015 »), l'institut national du cancer (INCA) reconnait une corrélation convaincante entre la consommation de fibres et la réduction de l'incidence du cancer colorectal et probable avec le cancer du sein.

Dès 1985, les chercheurs américains Graf et Eaton avaient montré que ce sont en fait les sels d'acide phytique présents dans les fibres de céréales qui sont à l'origine de l'effet anti-tumoral et non les fibres elles-mêmes (Graf E., Eaton J. W., Dietary supression of colonic cancer: Fiber or Phytate?, Cancer 1985, 56, 717-718). Les composés de l'acide phytique avaient longtemps été considérés comme des facteurs antinutritionnels en alimentation humaine car ils limitent l'absorption de certains minéraux essentiels tels que le calcium, le magnésium et le fer au niveau du tractus gastro-intestinal.

L'acide phytique (n° CAS : 83-86-3), également appelé acide myo-inositol hexaphosphorique, est une biomolécule présente sous forme de phytates associés à différents cations dans les fruits oléagineux, le pollen, les spores et dans les graines de nombreuses céréales et légumineuses où il représente jusqu'à 90 % des réserves totales en phosphore. L'acide phytique se retrouve principalement dans la couche à aleurone ou au niveau de l'embryon des graines.

L'acide phytique peut être extrait des matrices végétales sous forme de phytine. Le terme « phytine » désigne les sels d'acide phytique (ou phytates) qui comprennent essentiellement en tant que contre-ions Mg²⁺, Ca²⁺ et/ou K⁺. La phytine se retrouve généralement sous la forme d'un sel mixte de calcium et magnésium de l'acide phytique.

Néanmoins, des études récentes ont montré que l'acide phytique et la phytine peuvent exercer une large palette d'effets bénéfiques sur la santé humaine (cf. Bohn et al., Journal of Zhejang university Science B, 2008, 9(3) :165-191).

L'acide phytique et la phytine peuvent ainsi participer à l'abaissement du taux de cholestérol, à la normalisation de la glycémie et à la stimulation du système immunitaire. Ces composés peuvent également être utilisés pour prévenir la lithiase rénale, l'hyperpigmentation de la peau mais aussi le vieillissement prématuré des cellules grâce à leur activité antioxydante.

Il a été également montré que l'acide phytique était capable d'inhiber les principaux processus impliqués dans la cancérogénèse tels que la prolifération anormale des cellules et l'angiogenèse. L'acide phytique peut également induire l'apoptose et participer à la stimulation de la réponse immunitaire cellulaire.

Ainsi, les propriétés anti-tumorales de la phytine ont été démontrées par de nombreux essais précliniques montrant une réelle activité antiproliférative sur de nombreuses souches tumorales à des doses létales médianes DL₅₀ de 200 µM à 2,5 mM et sur des modèles animaux (cf. Vucenik I., Shamsuddine A. M., Cancer inhibition by inositol hexaphosphate (IP6) and inositol: from laboratory to clinic, Journal of nutrition 2003, 133: 3778S-3874S.

La publication Bacic I., Druzijanic N., Karlo R., Skific I., Jagic S., Efficacy of IP6 + inositol in the treatment of breast cancer patients receiving chemotherapy: prospective, randomized, pilot clinical study, Journal of Expérimental & Clinical Cancer Research 2010, 29:12, relate un essai clinique de faible envergure réalisé pendant six mois sur 14 patientes atteintes de cancer du sein ductal invasif. L'administration de phytine à forte dose (6 g/j), en complément d'une chimiothérapie, a préservé d'une cytopénie (déficit quantitatif en leucocytes et plaquettes), tout en améliorant la qualité de vie (réduction des effets secondaires), mais sans incidence sur la progression tumorale.

Le brevet européen EP 2452577 divulgue l'utilisation de phytine en association avec du myo-inositol et un composé de l'acide ascorbique pour la prévention des cancers et la stimulation du système immunitaire. En effet, il a été montré que l'association de la phytine avec le myo-inositol est capable de moduler certains processus biologiques impliqués dans la cancérogénèse tels que la prolifération anormale des cellules et l'angiogenèse, et il est suggéré que l'acide phytique et la vitamine C agissent en synergie pour stimuler la réponse immunitaire et prévenir le stress oxydant et donc le vieillissement cellulaire.

En conséquence, l'acide phytique et la phytine sont considérés comme des agents particulièrement intéressants pour le traitement mais également pour la prévention du cancer. Les dérivés pentaphosphoriques et tétraphosphoriques de l'inositol sont également connus pour présenter des propriétés anti-tumorales, le myo-inositol 1,3,4,5,6 pentakis phosphate étant même décrit comme possédant une activité anti-tumorale supérieure à celle de la phytine (Maffucci T. et al., Cancer Res. 2005, 65, 8339-49 : action anti-angiogénique en inhibant la voie PI3K/Akt à la concentration de 50 µM).

L'invention a pour objet une composition pharmaceutique comprenant du chlorure de magnésium et au moins un composé choisi parmi l'acide inositol hexaphosphorique, les acides inositol pentaphosphoriques, les acides inositol tétraphosphoriques ou l'un de leurs sels, en association avec un ou plusieurs excipients et/ou véhicules pharmaceutiquement acceptables.

Cette composition pharmaceutique est principalement prévue pour une utilisation à des fins thérapeutiques, de préférence pour une utilisation en vue du traitement et/ou de la prévention d'un cancer, en médecine humaine.

Il a en effet été démontré que la composition faisant l'objet de l'invention possédait une activité antiproliférative in vivo chez l'homme à l'encontre de tumeurs pancréatiques, très agressives, et qu'elle pouvait ainsi être utilisée pour le traitement ou à la prévention de l'apparition de cancers. De plus, la composition selon l'invention n'est pas cytotoxique in vivo à l'encontre de cellules saines aux doses pour laquelle elle possède une activité anticancéreuse.

### Description détaillée de l'invention

Certains composés de l'invention peuvent exister aussi bien sous forme solvatée que non solvatée, par exemple sous forme hydratée. En général, les formes solvatées sont équivalentes aux formes non solvatées et sont comprises dans la portée de la présente invention. Certains composés de la présente invention peuvent exister sous de multiples formes cristallines ou amorphes. En général, toutes les formes physiques sont équivalentes pour les utilisations envisagées par la présente invention et sont comprises dans la portée de la présente invention.

Certains des composés utilisés dans la composition selon l'invention possèdent un ou plusieurs centres asymétriques (optiques), de sorte que des stéréoisomères (énantiomères ou diastéroisomères) peuvent exister. Il est entendu que l'invention s'étend à tous les énantiomères et diastéroisomères de ces composés et à leurs mélanges, notamment les racémates. En d'autres termes, les composés utilisés dans la composition selon l'invention peuvent être utilisés sous la forme d'un énantiomère purifié ou sous la forme d'un mélange d'énantiomères. Les différents isomères peuvent être séparés selon des méthodes connues de l'homme du métier, notamment par chromatographie chirale, chromatographie liquide à haute performance (HPLC) ou par cristallisation fractionnée.

Dans la présente demande, on fera référence de façon générale à l'acide inositol hexaphosphorique et à ses sels, composés préférés selon l'invention, mais il est entendu que tous les modes de réalisation de l'invention concernant l'acide inositol hexaphosphorique et ses sels sont également applicables aux acides inositol pentaphosphoriques et aux acides inositol tétraphosphoriques ou à leurs sels.

Le premier constituant essentiel de la composition selon l'invention est un composé choisi parmi l'acide inositol hexaphosphorique, les acides inositol pentaphosphoriques, les acides inositol tétraphosphoriques ou l'un de leurs sels, de préférence l'acide inositol hexaphosphorique ou l'un de ses sels.

L'acide inositol hexaphosphorique ou l'un de ses sels est de préférence présent dans la composition selon l'invention sous la forme de son stéréoisomère acide myoinositol hexaphosphorique (acide phytique) ou l'un de ses sels. Il peut également être présent sous la forme d'un ou plusieurs autres stéréoisomères, notamment les stéréoisomères scyllo-, muco-, D-chiro- et néo-.

Les acides inositol pentaphosphoriques (notamment l'acide inositol 1,3,4,5,6-pentaphosphorique, de préférence en configuration myo) sont naturellement présents dans les hématies des oiseaux où ils interviennent comme effecteurs de libération de l'oxygène. Ils peuvent être également être obtenus par synthèse. Ils sont de préférence présents dans la composition selon l'invention sous la forme de leurs stéréoisomères acides myoinositol pentaphosphoriques. Ils peuvent également être présents sous la forme d'un ou plusieurs autres stéréoisomères, notamment les stéréoisomères scyllo-, muco-, D-chiro- et néo-.

Les acides inositol tétraphosphoriques sont de préférence présents dans la composition selon l'invention sous la forme de leurs stéréoisomères acides myo-inositol tétraphosphoriques. Ils peuvent également être présents sous la forme d'un ou plusieurs autres stéréoisomères, notamment les stéréoisomères scyllo-, muco-, D-chiro- et néo-. Ces composés sont de préférence choisis parmi l'acide inositol 1,3,4,5-tétraphosphorique et l'acide inositol 1,3,4,6-tétraphosphorique, tous deux de préférence en configuration myo.

Dans la présente demande, on fera référence de façon générale à l'acide phytique et à ses sels (phytine), mais il est entendu que tous les modes de réalisation de l'invention concernant l'acide phytique et ses sels sont applicables aux autres stéréoisomères de l'acide inositol hexaphosphorique et de ses sels. Cela signifie que l'expression « acide inositol hexaphosphorique » peut être substituée à l'expression « acide phytique » dans tous les modes de réalisation particuliers de l'invention. Il en va de même pour les expressions « acide inositol hexaphosphorique et ses sels » et « composés phytiques », désignant l'acide phytique et ses sels, et les expressions « sel de l'acide inositol hexaphosphorique » et « phytine ».

Dans la présente invention, l'acide inositol hexaphosphorique est préférentiellement utilisé sous la forme de l'un de ses sels, de préférence la phytine.

Au sens de la présente invention, un sel de l'acide inositol hexaphosphorique comprend un ou plusieurs contre-ions tels que Ca²⁺, Mg²⁺, K⁺, Na⁺, Fe²⁺, Fe³⁺, Co²⁺, Zn²⁺ et Cu²⁺. Dans un mode de réalisation préféré, ce sel correspond aux formes basiques de l'acide inositol hexaphosphorique, de préférence l'acide phytique (acide myo-inositol hexaphosphorique), associées principalement aux cations Ca²⁺ et/ou Mg²⁺ et/ou K⁺, mieux représente un sel mixte de calcium et magnésium de l'acide phytique, la phytine.

La phytine peut être obtenue à partir de graines de céréales. On peut citer comme source végétale adaptée à la préparation d'une phytine selon l'invention le blé, le maïs et le riz. A titre d'exemple, la phytine peut être obtenue à partir de son de blé, de son de riz et de l'embryon des graines de maïs.

Dans certains modes de réalisation, on utilise un sel de l'acide inositol hexaphosphorique ou une phytine obtenue à partir de graines de céréales et présentant un pourcentage massique en calcium de 0,05 % à 20 %, et/ou un pourcentage massique en magnésium de 3 % à 18 %, les pourcentages étant exprimés par rapport au poids total de phytine.

Lorsque la composition selon l'invention est liquide, de préférence aqueuse, l'acide inositol hexaphosphorique, les acides inositol pentaphosphoriques, les acides inositol tétraphosphoriques et leurs sels sont présents dans la composition à une teneur allant de 70 g/L à 270 g/L, de préférence de 80 g/L à 150 g/L, étant entendu que cette teneur est exprimée par rapport au volume total de la composition. Ces teneurs s'appliquent aussi individuellement à l'acide inositol hexaphosphorique et ses sels, les acides inositol pentaphosphoriques et ses sels et aux acides inositol tétraphosphoriques et leurs sels.

L'autre constituant essentiel de la composition selon l'invention est le chlorure de magnésium. Il peut être utilisé sous forme anhydre, hexahydratée, ou sous d'autres formes telles que le nigari, obtenu à partir de l'eau de mer.

Le chlorure de magnésium peut également être synthétisé *in situ* par traitement de sel de magnésium de l'acide inositol hexaphosphorique, d'acides inositol pentaphosphoriques, ou d'acides inositol tétraphosphoriques par l'acide chlorhydrique.

Généralement, l'acide inositol hexaphosphorique et ses sels d'une part, et le chlorure de magnésium d'autre part, sont présents dans un ratio massique allant de 4/1 à 16/1, de préférence de 6/1 à 12/1. Lorsque la composition selon l'invention est liquide, le chlorure de magnésium est présent de manière à ce que sa teneur soit comprise dans une gamme allant de 5 g/L à 30 g/L.

De préférence, l'acide inositol hexaphosphorique, les acides inositol pentaphosphoriques, les acides inositol tétraphosphoriques et leurs sels d'une part, et le chlorure de magnésium d'autre part (compté naturellement en tant que sel anhydre), sont présents dans un ratio massique allant de 2/1 à 16/1, de préférence de 4/1 à 12/1, mieux de 5/1 à 10/1.

Une composition pharmaceutique humaine selon l'invention peut aussi comprendre un ou plusieurs autres principes actifs différents de l'acide inositol hexaphosphorique, des acides inositol pentaphosphoriques, des acides inositol tétraphosphoriques ou l'un de leurs sels et du chlorure de magnésium, notamment pour augmenter son efficacité, y compris un ou plusieurs autres composés anticancéreux. Comme autres principes actifs susceptibles d'être inclus dans une composition pharmaceutique selon l'invention, on peut citer notamment des agents antihistaminiques, des agents anti-inflammatoires, des agents désinfectants ou encore des agents anesthésiques locaux.

Selon un mode de réalisation de l'invention, la composition selon l'invention contient uniquement comme principe actif anticancéreux l'acide inositol hexaphosphorique, les acides inositol pentaphosphoriques, les acides inositol tétraphosphoriques ou l'un de leurs sels, de préférence uniquement l'acide inositol hexaphosphorique ou l'un de ses sels.

Selon un autre mode de réalisation de l'invention, la composition selon l'invention contient un agent anticancéreux additionnel, tel qu'un taxane (par exemple le paclitaxel ou le docétaxel), la vinblastine, la vinorelbine, la vincristine, la bléomycine, le témozolomide, le 5-fluorouracile et/ou un inhibiteur d'angiogenèse (par exemple le bevacizumab).

Alternativement ou additionnellement, la composition selon l'invention peut être utilisée en association avec un autre traitement thérapeutique, notamment en association avec un traitement anticancéreux additionnel administré séparément, tel qu'un traitement au moyen de l'un des anticancéreux additionnels cités ci-dessus, ciblant par exemple la prolifération des cellules cancéreuses et/ou l'angiogenèse.

La composition peut comprendre au moins un composé supplémentaire présentant un intérêt physiologique ou pharmaceutique. Il peut s'agir d'un composé pouvant agir en combinaison avec les composés phytiques, par exemple, un composé capable de stimuler les défenses immunitaires ou encore d'un composé ayant une action distincte de celles de la phytine. Des exemples de tels composés sont les vitamines et leurs précurseurs, les acides aminés, les dérivés d'acides aminés tels que la taurine, les oligopeptides, les acides gras, les hormones, les flavonoïdes, les extraits de plantes, l'inositol et leurs mélanges. Ces composés supplémentaires sont décrits en détail dans le brevet EP 2452577, au nom du demandeur.

Le ou les composés supplémentaires à ajouter dans la composition peuvent être déterminés en fonction des effets physiologiques recherchés ou en fonction du public visé. A titre d'exemple, si l'on désire une activité antioxydante élevée, on pourra associer aux composés de l'acide inositol hexaphosphorique, par exemple, un composé choisi parmi la vitamine C, la vitamine E, la quercétine ou encore un extrait de thé vert.

Le ou les composés supplémentaires à but physiologique ou pharmaceutique sont généralement présents dans la composition, lorsqu'elle se trouve sous une forme liquide, à une teneur allant de 0,01 g/L à 900 g/L, de préférence, de 0,1 g/L à 400 g/L. La teneur d'un composé supplémentaire est à déterminer en fonction de la nature de ce composé et de l'effet recherché. Les composés supplémentaires étant couramment utilisés dans la préparation des compositions pharmaceutiques, l'homme du métier peut se référer aux doses usuelles utilisées dans l'état de la technique pour déterminer, pour chaque composé supplémentaire, la concentration à laquelle il doit être introduit dans la composition selon la présente invention.

Dans un mode de réalisation préféré selon l'invention, la composition pharmaceutique comprend en outre de l'inositol, de préférence du myo-inositol. Le myo-inositol (numéro de CAS : 87-89-8) correspond à la forme déphosphorylée de l'acide phytique et ne possède qu'une activité anti-tumorale modérée (Estensen, R. D., Wattenberg, L. W., Carcinogenesis 1993, 14 (9), 1975-1977). Cependant, ce composé agit comme une vitamine en présentant de nombreux effets bénéfiques améliorant l'état général du patient. Généralement, l'acide inositol hexaphosphorique, les acides inositol pentaphosphoriques, les acides inositol tétraphosphoriques et leurs sels d'une part, et l'inositol d'autre part (de préférence le myoinositol), sont présents dans un ratio massique allant de 1,5/1 à 8/1, de préférence de 2,5 à 6. Lorsque la composition selon l'invention est liquide, l'inositol est présent de manière à ce que sa teneur soit comprise dans une gamme allant de 15 g/L à 60 g/L.

De préférence, l'acide inositol hexaphosphorique et ses sels d'une part, et l'inositol d'autre part (de préférence le myo-inositol), sont présents dans un ratio massique allant de 1,5/1 à 8/1, de préférence de 2,5 à 6.

Les compositions pharmaceutiques comprenant un composé anticancéreux selon l'invention peuvent contenir un excipient et/ou un véhicule pharmaceutiquement acceptable, liquide ou solide, par exemple aqueux. De nombreux excipients et/ou véhicules pharmaceutiquement acceptables peuvent être utilisés, par exemple des solvants ou diluants ; l'eau, le cas échéant en mélange avec du propylène glycol ou du polyéthylène glycol, l'eau tamponnée, une solution saline, une solution de glycine et ses dérivés, une solution non aqueuse comprenant notamment des solvants tels que l'éthanol, la N-méthylpyrrolidone, le diméthylacétamide (DMA), le diméthylsulfoxyde (DMSO) et/ou le diméthyl formamide (DMF), ainsi que des agents nécessaires pour reproduire les conditions physiologiques comme par exemple des agents tampons et ajusteurs de pH, des tensioactifs comme le Solutol^{®} HS15, le Tween^{®} 80, l'acétate de sodium, le lactate de sodium, le chlorure de sodium, le chlorure de potassium, le chlorure de calcium, ou un véhicule tel que le Cremophor EL^{®}, cette liste n'étant pas limitative. De plus, la composition pharmaceutique peut être stérilisée par des techniques de stérilisation bien connues de l'homme du métier. On préfère utiliser dans la composition au moins un solvant aqueux et/ou au moins un tensioactif ou un acide carboxylique alpha-hydroxylé pour solubiliser l'acide inositol hexaphosphorique, les acides inositol pentaphosphoriques, les acides inositol tétraphosphoriques ou l'un de leurs sels et le chlorure de magnésium.

Selon un mode de réalisation préféré de l'invention, la composition comprend un solvant aqueux, pour solubiliser l'acide inositol hexaphosphorique, les acides inositol pentaphosphoriques, les acides inositol tétraphosphoriques ou leurs sels, lequel est de préférence combiné avec au moins un acide carboxylique alpha-hydroxylé. Selon un mode de réalisation préféré, la composition liquide selon l'invention est diluée dans une infusion de thé vert à température ambiante avant prise par le patient.

L'acide carboxylique alpha-hydroxylé agit à la fois en tant qu'agent solubilisant et agent stabilisateur. Il empêche, au moins partiellement, l'hydrolyse des groupements phosphate des dérivés inositol hexa, penta ou tétraphosphoriques, conduisant à des compositions pharmaceutiques liquides très stables dans le temps et pouvant être très concentrées en principes actifs.

Les acides carboxyliques alpha-hydroxylés utilisables dans la composition selon la présente invention sont décrits en détail dans le brevet EP 2452577, au nom du demandeur. Dans un mode de réalisation préféré, l'acide carboxylique alpha-hydroxylé est choisi parmi le groupe constitué par l'acide glycolique, l'acide gluconique, l'acide glucuronique, l'acide tartrique (notamment ses énantiomères L et D, et son diastéréoisomère méso), l'acide lactique, l'acide citrique, l'acide malique et leurs mélanges.

De préférence, la teneur en acide carboxylique alpha-hydroxylé dans la composition correspond à une quantité de groupe fonctionnel carboxyle allant de 3 mmol à 20 mmol par gramme de dérivés phytiques (acide phytique et phytine). Cette quantité assure une solubilisation et une stabilisation optimale de ces composés. La quantité préférentielle d'acide carboxylique alpha-hydroxylé à apporter varie donc en fonction du nombre de groupements carboxyle présents sur ledit acide carboxylique alpha-hydroxylé.

En tant que véhicules, adjuvants ou excipients inertes, non toxiques, pharmaceutiquement acceptables, on peut aussi citer à titre indicatif et non limitatif les agents solubilisants autres que les solvants, les conservateurs (afin d'éviter notamment la contamination bactérienne ou fongique lors du conditionnement), les agents mouillants, les émulsifiants, les agents dispersants, les liants, les agents gonflants, les agents désintégrants, les agents d'encapsulation, les retardants, les lubrifiants, les absorbants, les agents de suspension, les colorants, les agents texturants, les arômes, les stabilisants, les agents épaississants etc. De tels composés sont par exemple le carbonate de magnésium, le stéarate de magnésium, le talc, le lactose, la pectine, la dextrine, l'amidon, la gélatine, des matériaux cellulosiques, du beurre de cacao, etc.

Des véhicules et des excipients (ou adjuvants) physiologiquement acceptables sont aussi décrits dans l'ouvrage intitulé "Handbook of Pharmaceutical Excipients," Seconde édition, American Pharmaceutical Association, 1994.

De préférence, la masse de l'extrait sec d'une composition liquide selon l'invention représente de 10 à 30 % de la masse totale de cette composition, mieux de 15 à 25 %. Par extrait sec, on entend selon la présente invention les matières solides restantes après évaporation des solvants et composés volatils dans un four à 100-110°C, éventuellement sous vide.

De manière générale, l'acide inositol hexaphosphorique, les acides inositol pentaphosphoriques, les acides inositol tétraphosphoriques et ses sels représentent de 25 à 55 % de la masse de l'extrait sec de la composition, et avantageusement de 30 à 50 %.

De manière générale, l'acide inositol hexaphosphorique et ses sels représentent de 25 à 55 % de la masse de l'extrait sec de la composition, et avantageusement de 30 à 50 %, mieux de 35 à 45 %.

De manière générale, le chlorure de magnésium représente de 1 à 10 % de la masse de l'extrait sec de la composition, et avantageusement de 2 à 8 %, mieux de 3 à 7 %.

De manière générale, l'inositol (de préférence le myo-inositol) représente de 4 à 20 % de la masse de l'extrait sec de la composition, et avantageusement de 5 à 18 %, mieux de 7 à 15 %.

De manière générale, les excipients représentent de 25 à 55 % de la masse de l'extrait sec de la composition, et avantageusement de 30 à 50 %, mieux de 35 à 45 %.

De manière générale, une composition pharmaceutique selon l'invention comprend de 1 % à 99 en poids, et avantageusement de 50 % à 97 % en poids, mieux de 75 à 95 % d'un excipient et/ou véhicule (ou diluant) ou d'une combinaison d'excipients et/ou véhicules pharmaceutiquement acceptables.

Une composition pharmaceutique selon l'invention se présente indifféremment sous une forme solide (poudre généralement hydrosoluble, de préférence sous forme sèche, c'est-à-dire en l'absence de solvant) ou sous une forme liquide. Sous la forme liquide, on préférera une composition pharmaceutique sous la forme d'une suspension aqueuse ou d'une solution aqueuse, de préférence une solution aqueuse, ou encore sous la forme d'une émulsion eau-dans-huile ou huile-dans-eau ou bien encore d'une suspension colloïdale en présence par exemple de phospholipides, en vue d'accroître la biodisponibilité. Les formulations dans lesquelles les composés phytiques sont dans un état solubilisé sont préférées, mais une formulation de la composition sous forme de poudre (pour une utilisation finale de préférence sous forme liquide) est également intéressante car elle évite les problèmes de stabilité qui peuvent être rencontrés en milieu liquide. Ainsi, la composition pharmaceutique peut notamment être conditionnée par exemple en dose journalière, à dissoudre dans un milieu aqueux avant la prise par le patient.

La forme préférée pour la composition selon l'invention est la solution aqueuse, car elle assure une biodisponibilité en composés phytiques plus élevée que les formulations solides (gélule, comprimé...). On entend par « solution aqueuse » une solution comprenant en tant que solvant principal l'eau, c'est-à-dire une solution comprenant au moins 50 % en volume d'eau, par rapport au volume total de ladite solution, de préférence au moins 90 %. Cette solution aqueuse peut comprendre de 0,1 % à 10 % en poids d'un solvant miscible à l'eau, notamment l'éthanol ou un solvant aprotique polaire. Dans certains modes de réalisation, la solution aqueuse comprend uniquement à titre de solvant de l'eau. La composition aqueuse selon l'invention a généralement un pH allant de 3 à 7,5, typiquement de 3 à 5.

La préparation de solutions aqueuses de phytine est décrite en détail dans le brevet EP 2452577, au nom du demandeur.

Dans certains modes de réalisation, la composition pharmaceutique est exempte de tout agent texturant tel que les agents épaississants et les agents gélifiants. En effet, la présence de ces agents peut être préjudiciable au caractère liquide et à l'homogénéité de la composition. En particulier, la composition ne contient pas, dans ce mode de réalisation, de composés gélifiants tels que l'agar-agar, l'amidon, les sels d'alginate, le carraghénane et les gélatines animales.

Lorsque l'on prépare une composition solide sous forme de comprimés, on mélange les ingrédients actifs principaux avec un véhicule pharmaceutique tel que la gélatine, l'amidon, le lactose, le stéarate de magnésium, le talc, la gomme arabique ou analogues.

On peut enrober les comprimés de saccharose ou d'autres matières premières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant les ingrédients actifs avec un diluant et en versant le mélange obtenu dans des gélules molles ou dures.

Les principes actifs peuvent être formulés également sous forme de microcapsules, éventuellement avec un ou plusieurs supports ou additifs.

Une composition pharmaceutique sous forme de sirop ou d'élixir peut contenir les ingrédients actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir les ingrédients actifs en mélange avec des agents de dispersion ou des agents mouillants ou des agents de mise en suspension, comme la polyvinylpyrrolidone, de même qu'avec des édulcorants ou des correcteurs de goût.

De manière générale, pour fabriquer une composition pharmaceutique conforme à l'invention, l'homme du métier peut avantageusement se référer à la 9^{ème} édition de la Pharmacopée Européenne publiée en juillet 2016, ou à la dernière édition de la Pharmacopée des Etats-Unis d'Amérique (U.S. Pharmacopeia, notamment l'édition USP 40-NF 35).

Des techniques de préparation de compositions pharmaceutiques selon l'invention peuvent être aisément retrouvées par l'homme du métier, par exemple dans l'ouvrage Remington's Pharmaceutical Sciences, 17th édition, Mack Publishing Company, Easton, Pen, USA, 1985).

Parmi les compositions pharmaceutiques selon l'invention, on pourra citer plus particulièrement celles qui conviennent pour l'administration orale, topique, parentérale, nasale, intraveineuse, percutanée (transcutanée), sous-cutanée, rectale, perlinguale ou respiratoire et notamment les comprimés simples ou dragéifiés, les comprimés sublinguaux, les gélules, les tablettes, les suppositoires, les crèmes, les pommades, les gels dermiques, les ampoules buvables ou injectables ou d'autres préparations liquides.

La composition pharmaceutique de la présente invention peut être utilisée pour une administration de manière prophylactique et/ou thérapeutique. Ainsi les principes actifs selon la présente invention sont préparés sous une forme adaptée au type d'administration choisi, par exemple sous forme liquide ou sous forme lyophilisée.

Pour une administration par voie orale, une composition pharmaceutique selon l'invention peut de présenter sous la forme de comprimés, de gélules, de capsules, de dragées, de sirops, de suspensions, de solutions, de poudres, de granulés, d'émulsions, de suspensions de microsphères ou de nanosphères, de suspensions de vésicules lipidiques ou de vésicules à base de divers polymères.

Spécifiquement, pour une administration orale, une composition pharmaceutique selon l'invention peut se présenter sous la forme de comprimés qui peuvent être fabriqués à partir de compositions solides en combinaison avec divers excipients tels que la cellulose microcristalline, le citrate de sodium, le carbonate de calcium, le phosphate dicalcique, ou la glycine. On peut utiliser divers agents désintégrants tels que l'amidon (de maïs, de pommes de terre, de tapioca, etc.), de l'acide alginique ou encore un silicate. On peut utiliser des agents liants tels que la polyvinylpyrrolidone, le saccharose, la gélatine, ou encore l'acacia. On peut utiliser des agents lubrifiants tels que le stéarate de magnésium, le lauryl sulfate de sodium, ou encore le talc. De telles compositions solides, sous forme d'une poudre, peuvent être utilisées pour la fabrication de capsules de gélatine. Pour des compositions solides, on peut aussi utiliser du lactose ou encore un polyéthylène glycol de haut poids moléculaire.

Pour fabriquer des compositions liquides pour administration orale, l'acide inositol hexaphosphorique ou l'un de ses sels et le chlorure de magnésium peuvent être combinés avec divers agents édulcorants, agents d'arôme, agents colorants, éventuellement avec également des agents émulsifiants ou des agents de suspension, en combinaison avec des agents diluants tels que l'eau, l'éthanol, un propylène glycol, de la glycérine ou toute combinaison de ces agents excipients.

Pour une administration par voie parentérale, une composition pharmaceutique selon l'invention peut se présenter sous la forme de solutions ou de suspensions pour perfusion ou injection. Ainsi, on peut utiliser en particulier des solutions huileuses ou aqueuses ou encore des suspensions, des émulsions, ou des implants y compris des suppositoires. Par exemple, les principes actifs de la composition peuvent être dispersés dans un véhicule liquide tel qu'un liquide salin physiologique ou encore une solution saline contenant 5 % en poids de dextrose, qui sont classiquement utilisées pour la préparation de formulations pharmaceutiques injectables.

Pour une administration par voie entérale, on peut utiliser des compositions à libération contrôlée, par exemple des compositions dans lesquelles les principes actifs de la composition sont protégés du milieu extérieur par une pluralité de couches de revêtement qui se dégradent de manière différente, par exemple au contact d'un milieu neutre ou basique (couches de revêtement gastro-résistantes) ou au contact d'un milieu aqueux (couches de revêtement comprenant des polymères solubles ou qui se dégradent dans l'eau).

La posologie varie selon le sexe, l'âge, le poids et l'état général du patient, selon la voie d'administration, selon le type de cancer, l'état de progression du cancer, en particulier selon que des métastases ont été détectées ou non chez le patient. La posologie peut également varier selon le type du ou des traitement(s) anti-cancéreux associé(s). Elle est déterminée au cas par cas, sous contrôle médical.

De manière générale, on utilise l'acide inositol hexaphosphorique et ses sels (et/ou les acides inositol pentaphosphoriques, les acides inositol tétraphosphoriques et leurs sels) dans des quantités allant de préférence de 0,01 mg/kg de poids corporel du patient ou de l'animal, à 1 g/kg de poids corporel du patient ou de l'animal par 24 heures, de préférence de 50 à 500 mg/Kg, en une ou plusieurs prises, typiquement deux prises journalières. De préférence, ladite quantité est au moins égale à 10 mg/kg, mieux 75 mg/kg. De préférence, ladite quantité est au plus égale à 500 mg/kg, mieux 250 mg/kg.

A titre indicatif, en traitement d'attaque, on peut prévoir 6 à 8 g/jour de dérivés phosphoriques de l'inositol selon l'invention et 1,2 à 1,6 g/jour de MgCl₂, à prendre en deux prises entre les repas ; en traitement de soutien à maintenir en fonction des résultats de l'analyse des marqueurs tumoraux, on peut prévoir 3 à 4 g/jour de dérivés phosphoriques de l'inositol selon l'invention et 0,8 à 1,2 g/jour de MgCl₂, à prendre en une seule prise; en traitement de prévention primaire ou tertiaire pour diminuer le risque de récidive, on peut prévoir 1 à 2 g/jour de dérivés phosphoriques de l'inositol selon l'invention et 0,4 à 0,8 g/jour de MgCl₂, à prendre en une seule prise.

La composition selon la présente invention peut être conditionnée dans un contenant multi-dose ou dans un contenant uni-dose. De manière préférée, elle est conditionnée en unités de dose, par exemple sous la forme de dosettes ou d'ampoules uni-doses sécables.

Comme cela a déjà été mentionné précédemment dans la présente description, la composition selon l'invention possède une activité contre les cellules cancéreuses de type antiproliférative mais non cytotoxique, dont il découle un avantage très important qui est l'absence ou la quasi-absence d'effets secondaires d'un traitement au moyen de ladite composition. En effet, ses constituants ne sont pas toxiques et pourraient tout au plus entraîner un risque d'anémie sur le long terme dû à la complexation du fer par les dérivés phosphoriques de l'inositol.

Le mécanisme d'action précis d'une composition selon l'invention n'est pas connu. Sans vouloir être lié par une quelconque théorie, il est possible que le chlorure de magnésium agisse en tant qu'anti-inflammatoire ou activateur des défenses immunitaires non-spécifique, et que les composés phosphoriques de l'inositol lèvent l'inhibition de l'apoptose et limitent l'angiogenèse des cellules cancéreuses. Egalement, la re-différenciation des cellules cancéreuses avec retour au phénotype normal pourrait être impliquée, comme cela a été observé sur des cultures de cellules végétales réalisées en présence d'acide phytique (Parc G., Rembur J., Rech P., Chriqui D., Plant Cell Rep. 2007, 26, 145-52). Ces composés phosphoriques de l'inositol pourraient également intervenir par séquestration du fer plasmatique ou en neutralisant des composés indispensables à la progression tumorale tels que des polyamines.

Les effets de la composition selon l'invention résultent d'une synergie entre le chlorure de magnésium et les composés phosphoriques de l'inositol ou leurs sels (cf. partie expérimentale). Le chlorure de magnésium n'a pas d'action anti-tumorale. Il a été initialement utilisé au cours de la première guerre mondiale par le Professeur Pierre Delbet comme antiseptique pour désinfecter les plaies de guerre. Jusque dans les années 1960, il a été utilisé avec succès pour traiter des affections bactériennes ou virales aussi graves que la diphtérie ou la poliomyélite.

Contrairement aux anticorps monoclonaux dirigés vers des cibles spécifiques (gènes défectueux ou antigènes tumoraux spécifiques d'un type de tumeur), la composition selon l'invention, de par la nature de ses constituants, est efficace sur de nombreux types de tumeurs.

Dans la présente demande, les cellules cancéreuses désignent des cellules possédant des caractéristiques typiques de cellules provoquant le cancer, telles qu'une prolifération incontrôlée, immortalité, potentiel métastatique, croissance rapide et grande vitesse de prolifération, et certaines caractéristiques morphologiques spécifiques. Les cellules cancéreuses se présentent souvent sous la forme d'une tumeur, mais de telles cellules peuvent exister seules à l'intérieur du corps, ou peuvent être des cellules cancéreuses non tumorigènes, telles que des cellules leucémiques. Les cellules cancéreuses peuvent être associées à de nombreux types de cancers, comprenant sans limitation, la leucémie, l'ampullome vatérien, un lymphome, un mélanome, un neuroblastome, le cancer du foie, des ovaires, du cerveau, du poumon (en particulier un cancer du poumon non à petites cellules), du colon, du sein, du pancréas, de la prostate, du testicule, de l'œsophage, de la vésicule biliaire, de l'intestin grêle, de l'utérus, du col de l'utérus, du rein, de l'estomac, de la vessie, un cancer cérébrospinal, un cancer colorectal, un glioblastome multiforme, de préférence le cancer du pancréas. Les compositions pharmaceutiques de l'invention peuvent être utilisées pour le traitement thérapeutique d'au moins l'un des cancers cités ci-dessus, notamment un cancer choisi parmi le cancer du sein, l'ampullome vatérien, le cancer du pancréas, le cancer du foie, le cancer de la vésicule biliaire, le cancer de l'intestin grêle et le cancer colorectal.

L'invention a donc pour objet une composition pharmaceutique telle que définie précédemment, pour une utilisation à des fins thérapeutiques, c'est-à-dire en tant que médicament à usage humain ou vétérinaire, en particulier pour une utilisation en vue du traitement et/ou de la prévention d'un cancer, métastatique ou primaire.

L'invention concerne aussi une méthode de traitement thérapeutique pour prévenir et/ou traiter le développement d'un cancer chez un patient nécessitant un tel traitement, ladite méthode comprenant une étape au cours de laquelle on administre au patient, une quantité thérapeutiquement efficace d'une composition telle que définie dans la présente description, soit seule, soit en mélange avec un ou plusieurs excipients et/ou véhicules pharmaceutiquement acceptables.

L'invention peut être mise en oeuvre en combinaison avec d'autres modalités de traitements, telles que l'hormonothérapie, la chirurgie, la cryothérapie, l'hyperthermie, la radiothérapie, une chimiothérapie additionnelle, etc. Avantageusement, le traitement selon l'invention peut être associé avec un autre traitement ciblant la prolifération des cellules cancéreuses et/ou l'angiogenèse.

L'invention est en outre illustrée, sans pour autant être limitée, par les exemples suivants.

### Partie expérimentale

La composition massique de la phytine utilisée, fournie par Tsuno Fine Rice Chemicals (Wakayama, Japon) est la suivante : phosphore 23,3 %, calcium 18,5 %, magnésium 4,8 %. Le chlorure de magnésium anhydre, le myo-inositol et l'acide citrique monohydraté sont de qualité Codex et fournis par Coger (79 rue des Morillons 75015 Paris).

Une composition selon l'invention a été administrée à un patient volontaire ayant développé une tumeur cancéreuse et a permis de démontrer son efficacité anti-tumorale in vivo.

### 1. Etudes préalables

Toxicité aigüe : Les solutions de phytine ne présentent aucun effet secondaire visible après administration orale à la dose unique de 2240 g/kg chez le rat de la lignée Wistar mâle et femelle. L'absence de toxicité chez l'homme du chlorure de magnésium était déjà connue.

Activité antiproliférative des solutions de phytine : Des essais sur des souches cellulaires cancéreuses ont été réalisés sur les souches suivantes : DU-145 (cancer prostatique humain), EMT-6 (carcinome mammaire de souris), SW-480 (adénocarcinome de côlon humain), MCF-7 (lignée humaine tumorale mammaire ER positive). Les concentrations DL₅₀ sur ces souches se situent entre 250 et 500 µM. Ces concentrations sont supérieures à celles d'anti-tumoraux classiques (doxorubicine, étoposide, méthotrexate, 5- fluorouracile), mais il est important de souligner que les solutions de phytine ne sont pas toxiques.

### 2. Patient et traitement

Le patient est un homme âgé de 90 ans, qui présentait des antécédents d'adénome de la prostate, de dyslipidémie, d'hypertension artérielle, d'arythmie cardiaque et fibrillation auriculaire et de carcinome baso-cellulaire. Son état général s'est dégradé avec fatigue intense, troubles digestifs, sarcopénie et perte de poids de 6 kg les trois derniers mois avant son admission aux urgences pour ictère suite à des douleurs abdominales, nausées, vomissements et syncope.

Préalablement à son admission aux urgences, le patient a consommé régulièrement, avec une visée préventive et pendant 5 ans, sous forme de complément alimentaire buvable, 1,4 g/jour de phytine solubilisée par l'acide citrique.

Un scanner a montré une dilatation des voies biliaires et du canal de Wirsung. Une écho- endoscopie a confirmé la présence d'un ampullome vatérien (tumeur maligne de l'ampoule hépatopancréatique) à développement intra-ampullaire d'étiologie néoplasique. La solution habituelle pour ce type de pathologie est une duodénopancréatectomie céphalique (ablation chirurgicale de la tête du pancréas, de la totalité du duodénum, de la vésicule biliaire et souvent du tiers inférieur de l'estomac).

Le patient a été traité pendant 66 jours au moyen de deux doses journalières de traitement selon l'invention, matin et après-midi entre les repas.

La préparation d'une dose de traitement est réalisée comme suit : un mélange composé de 3,2 g de phytine et de 0,88 g de myo-inositol sont dispersés dans 30 mL d'eau. 3,4 g d'acide citrique monohydraté sont ajoutés dans le but de dissoudre la phytine, ainsi qu'un comprimé de 392 mg de chlorure de magnésium anhydre (soit 100 mg de magnésium). Après dissolution complète, cette préparation est diluée avec une infusion de thé vert refroidie avant d'être ingérée à température ambiante.

### 3. Résultats de l'essai clinique

A l'issue du traitement de 66 jours, une scintigraphie au ¹⁸FDG a été réalisée (Tomographie par émission de positons - TEP-SCAN - sur Siemens Biograph mCTFLOW, injection de 137MBq de ¹⁸FDG, acquisition TEP couplée à une tomodensitométrie de correction d'atténuation à faible dose sans injection de produit de contraste). Elle indique une absence de foyer discernable du bruit de fond au niveau du pancréas et du confluent duodéno-pancréatique. Il n'y a pas d'argument non plus pour une atteinte secondaire ganglionnaire, viscérale ou osseuse.

Une IRM hépatique et cholangio-IRM réalisée quatre jours plus tard indique une absence de processus expansif au niveau du pancréas céphalique ou au niveau ampullaire.

Une écho-endoscopie réalisée sous anesthésie générale 40 jours plus tard à l'aide de la sonde d'écho-endoscopie Olympus confirme la disparition de l'ampullome vatérien.

Le fait que le traitement selon l'invention soit actif sur le cancer du pancréas, l'un des cancers dont le pronostic vital est le plus sombre, suggère que ce traitement devrait être efficace sur de nombreux types de cancers. Le cancer du pancréas est un cancer très agressif qui résiste à toutes les chimiothérapies. La survie après la détection d'un cancer du pancréas n'excède pas généralement 5 mois.

Ces résultats cliniques remarquables étaient tout à fait inattendus compte tenu des propriétés connues des dérivés phytiques d'une part et du chlorure de magnésium d'autre part, et résultent manifestement d'une synergie in vivo. Effectivement, l'effet résultant de leur association est sans commune mesure avec la somme des effets constatés lorsqu'ils opèrent indépendamment.

D'une part, le fait que le patient ait consommé régulièrement de la phytine antérieurement au diagnostic d'ampullome vatérien confirme que celle-ci n'a pas d'action anti-tumorale chez l'homme lorsqu'elle est utilisée en l'absence de chlorure de magnésium.

D'autre part, les dérivés magnésiens n'ont jamais permis de traiter le cancer. A ce sujet, les Docteurs J. Sal et Y. Donadieu ont déclaré : « Il est bien évident qu'il est impossible de traiter un cancer, quel qu'il soit, par un seul traitement magnésien » (Le Magnésium : thérapeutique naturelle, Ed. Maloine, Paris, 1986, p. 66).

Ces résultats sont à rapprocher des données comparatives suivantes, disponibles dans la littérature. La phytine et l'inositol, utilisés en complément d'une chimiothérapie, à dose identique à celle du traitement mis en jeu ci-dessus mais en l'absence de chlorure de magnésium, n'entrainent qu'une réduction des effets secondaires (Journal of Experimental & Clinical Cancer Research 2010, 29:12). L'inventeur a également constaté qu'in vitro, le chlorure de magnésium n'améliorait pas l'activité anti-tumorale de la phytine solubilisée par l'acide citrique sur la lignée de cellules tumorales mammaires MCF-7 (aucune modification de la concentration inhibitrice médiane IC₅₀), et que le myo-inositol n'avait pas d'action anti-tumorale in vitro sur la souche MCF-7.

## Revendications

1. Composition pharmaceutique pour son utilisation dans le traitement et/ou la prévention d'un cancer chez l'homme, comprenant :
- au moins un composé choisi parmi l'acide inositol hexaphosphorique, les acides inositol pentaphosphoriques, les acides inositol tétraphosphoriques ou l'un de leurs sels, et
- du chlorure de magnésium, en association avec un ou plusieurs excipients et/ou véhicules pharmaceutiquement acceptables.

2. Composition pharmaceutique pour son utilisation selon la revendication 1, **caractérisée en ce qu'**elle comprend de l'acide inositol hexaphosphorique ou l'un de ses sels présent sous la forme de son stéréoisomère acide myo-inositol hexaphosphorique ou l'un de ses sels.

3. Composition pharmaceutique pour son utilisation selon l'une quelconque des revendications précédentes, **caractérisée en ce qu'**elle comprend de l'acide inositol hexaphosphorique présent sous la forme d'un sel mixte de calcium et de magnésium.

4. Composition pharmaceutique pour son utilisation selon l'une quelconque des revendications précédentes, **caractérisée en ce qu'**elle comprend en outre de l'inositol.

5. Composition pharmaceutique pour son utilisation selon la revendication 4, **caractérisée en ce que** l'inositol est employé sous forme de myo-inositol.

6. Composition pharmaceutique pour son utilisation selon la revendication 4 ou 5, **caractérisée en ce que** l'acide inositol hexaphosphorique, les acides inositol pentaphosphoriques, les acides inositol tétraphosphoriques et leurs sels d'une part, et l'inositol d'autre part, sont présents dans un ratio massique allant de 1,5/1 à 8/1.

7. Composition pharmaceutique pour son utilisation selon l'une quelconque des revendications précédentes, **caractérisée en ce qu'**elle comprend en outre au moins un acide carboxylique alpha-hydroxylé.

8. Composition pharmaceutique pour son utilisation selon la revendication 7, **caractérisée en ce que** l'acide carboxylique alpha-hydroxylé est choisi parmi l'acide lactique, l'acide tartrique, l'acide glycolique, l'acide gluconique, l'acide glucuronique, l'acide citrique et l'acide malique.

9. Composition pharmaceutique pour son utilisation selon l'une quelconque des revendications précédentes, **caractérisée en ce qu'**elle comprend en outre un solvant aqueux.

10. Composition pharmaceutique pour son utilisation selon l'une quelconque des revendications précédentes, **caractérisée en ce qu'**elle se présente sous forme de solution aqueuse, l'acide inositol hexaphosphorique, les acides inositol pentaphosphoriques, les acides inositol tétraphosphoriques et leurs sels étant présents à une teneur allant de 70 g/L à 270 g/L.

11. Composition pharmaceutique pour son utilisation selon l'une quelconque des revendications 1 à 8 **caractérisée en ce qu'**elle se présente sous forme de poudre.

12. Composition pharmaceutique pour son utilisation selon l'une quelconque des revendications précédentes, **caractérisée en ce que** l'acide inositol hexaphosphorique, les acides inositol pentaphosphoriques, les acides inositol tétraphosphoriques et leurs sels d'une part, et le chlorure de magnésium d'autre part, sont présents dans un ratio massique allant de 2/1 à 16/1.

13. Composition pharmaceutique pour son utilisation selon l'une quelconque des revendications précédentes, **caractérisée en ce que** le cancer est choisi parmi le cancer du sein, l'ampullome vatérien, le cancer du pancréas, le cancer du foie, le cancer de la vésicule biliaire, le cancer de l'intestin grêle et le cancer colorectal.

14. Composition pharmaceutique pour son utilisation selon l'une quelconque des revendications précédentes, **caractérisée en ce qu'**elle comprend en outre un extrait de thé vert.

15. Composition pharmaceutique pour son utilisation selon l'une quelconque des revendications précédentes, pour une utilisation en vue du traitement et/ou de la prévention du cancer du pancréas.

## Patentansprüche

1. Pharmazeutische Zusammensetzung zur Verwendung bei der Behandlung und/oder Vorbeugung von Krebs beim Menschen, umfassend:
- mindestens eine Verbindung, ausgewählt aus Inositolhexaphosphorsäure, Inositolpentaphosphorsäuren, Inositoltetraphosphorsäuren oder einem ihrer Salze, und
- Magnesiumchlorid, in Kombination mit einem oder mehreren pharmazeutisch annehmbaren Exzipienten und/oder Vehikeln.

2. Pharmazeutische Zusammensetzung zur Verwendung nach Anspruch 1, **dadurch gekennzeichnet, dass** sie Inositolhexaphosphorsäure oder eines ihrer Salze umfasst, die in Form ihres Stereoisomers Myoinositolhexaphosphorsäure oder eines ihrer Salze vorliegt.

3. Pharmazeutische Zusammensetzung zur Verwendung nach einem der vorhergehenden Ansprüche, **dadurch gekennzeichnet, dass** sie Inositolhexaphosphorsäure umfasst, die in Form eines gemischten Calcium- und Magnesiumsalzes vorliegt.

4. Pharmazeutische Zusammensetzung zur Verwendung einem der vorhergehenden Ansprüche, **dadurch gekennzeichnet, dass** sie außerdem Inositol umfasst.

5. Pharmazeutische Zusammensetzung zur Verwendung nach Anspruch 4, **dadurch gekennzeichnet, dass** das Inositol in Form von Myoinositol eingesetzt wird.

6. Pharmazeutische Zusammensetzung zur Verwendung nach Anspruch 4 oder 5, **dadurch gekennzeichnet, dass** die Inositolhexaphosphorsäure, die Inositolpentaphosphorsäuren, die Inositoltetraphosphorsäuren und ihre Salze einerseits und Inositol andererseits in einem Massenverhältnis im Bereich von 1,5/1 bis 8/1 vorliegen.

7. Pharmazeutische Zusammensetzung zur Verwendung nach einem der vorhergehenden Ansprüche, **dadurch gekennzeichnet, dass** sie außerdem mindestens eine alpha-Hydroxycarbonsäure umfasst.

8. Pharmazeutische Zusammensetzung zur Verwendung nach Anspruch 7, **dadurch gekennzeichnet, dass** die alpha-Hydroxycarbonsäure aus Milchsäure, Weinsäure, Glykolsäure, Gluconsäure, Glucuronsäure, Zitronensäure und Äpfelsäure ausgewählt ist.

9. Pharmazeutische Zusammensetzung zur Verwendung nach einem der vorhergehenden Ansprüche, **dadurch gekennzeichnet, dass** sie außerdem ein wässriges Lösungsmittel umfasst.

10. Pharmazeutische Zusammensetzung zur Verwendung nach einem der vorhergehenden Ansprüche, **dadurch gekennzeichnet, dass** sie in Form einer wässrigen Lösung vorliegt, wobei die Inositolhexaphosphorsäure, die Inositolpentaphosphorsäuren, die Inositoltetraphosphorsäuren und ihre Salze in einem Gehalt im Bereich von 70 g/l bis 270 g/l vorliegen.

11. Pharmazeutische Zusammensetzung zur Verwendung nach einem der Ansprüche 1 bis 8, **dadurch gekennzeichnet, dass** sie in Pulverform vorliegt.

12. Pharmazeutische Zusammensetzung zur Verwendung nach einem der vorhergehenden Ansprüche, **dadurch gekennzeichnet, dass** die Inositolhexaphosphorsäure, die Inositolpentaphosphorsäuren, die Inositoltetraphosphorsäuren und ihre Salze einerseits und Magnesiumchlorid andererseits in einem Massenverhältnis im Bereich von 2/1 bis 16/1 vorliegen.

13. Pharmazeutische Zusammensetzung zur Verwendung nach einem der vorhergehenden Ansprüche, **dadurch gekennzeichnet, dass** der Krebs aus Brustkrebs, Karzinom der Ampulla Vateri, Bauchspeicheldrüsenkrebs, Leberkrebs, Gallenblasenkrebs, Dünndarmkrebs und Kolorektalkrebs ausgewählt ist.

14. Pharmazeutische Zusammensetzung zur Verwendung nach einem der vorhergehenden Ansprüche, **dadurch gekennzeichnet, dass** sie außerdem einen Grüntee-Extrakt umfasst.

15. Pharmazeutische Zusammensetzung zur Verwendung nach einem der vorhergehenden Ansprüche zur Verwendung bei der Behandlung und/oder Vorbeugung von Bauchspeicheldrüsenkrebs.

## Claims

1. Pharmaceutical composition for its use in the treatment and/or prevention of cancer in humans, comprising:
- at least one compound selected from inositol hexaphosphoric acid, inositol pentaphosphoric acids, inositol tetraphosphoric acids or one of their salts, and
- magnesium chloride, in combination with one or more pharmaceutically acceptable excipients and/or vehicles.

2. Pharmaceutical composition for its use according to claim 1, wherein the pharmaceutical composition comprises inositol hexaphosphoric acid or a salt thereof, present in the form of its stereoisomer myo-inositol hexaphosphoric acid or a salt thereof.

3. Pharmaceutical composition for its use according to any one of the preceding claims, wherein the pharmaceutical composition comprises inositol hexaphosphoric acid, present in the form of a mixed salt of calcium and magnesium.

4. Pharmaceutical composition for its use according to any one of the preceding claims, wherein the pharmaceutical composition further comprises inositol.

5. Pharmaceutical composition for its use according to claim 4, wherein inositol is used under the form of myo-inositol.

6. Pharmaceutical composition for its use according to claim 4 or 5, wherein inositol hexaphosphoric acid, inositol pentaphosphoric acids, inositol tetraphosphoric acids and salts thereof on the one hand, and inositol on the other hand, are present in a mass ratio ranging from 1.5/l to 8/l.

7. Pharmaceutical composition for its use according to any one of the preceding claims, wherein the pharmaceutical composition further comprises at least one alpha-hydroxylated carboxylic acid.

8. Pharmaceutical composition for its use according to claim 7, wherein the alpha-hydroxylated carboxylic acid is selected from lactic acid, tartaric acid, glycolic acid, gluconic acid, glucuronic acid, citric acid and malic acid.

9. Pharmaceutical composition for its use according to any one of the preceding claims, wherein the pharmaceutical composition further comprises an aqueous solvent.

10. Pharmaceutical composition for its use according to any one of the preceding claims, wherein the pharmaceutical composition is in the form of an aqueous solution, inositol hexaphosphoric acid, inositol pentaphosphoric acids, inositol tetraphosphoric acids and salts thereof being present in an amount of from 70 g/L to 270 g/L.

11. Pharmaceutical composition for its use according to any one of claims 1 to 8, wherein the pharmaceutical composition is in the form of a powder.

12. Pharmaceutical composition for its use according to any one of the preceding claims, wherein inositol hexaphosphoric acid, inositol pentaphosphoric acids, inositol tetraphosphoric acids and salts thereof on the one hand, and magnesium chloride on the other hand, are present in a mass ratio ranging from 2/l to 16/l.

13. Pharmaceutical composition for its use according to any one of the preceding claims, wherein the cancer is selected from breast cancer, periampullary cancer, pancreatic cancer, liver cancer, gallbladder cancer, small intestine cancer and colorectal cancer.

14. Pharmaceutical composition for its use according to any one of the preceding claims, wherein the pharmaceutical composition further comprises a green tea extract.

15. Pharmaceutical composition for its use according to any one of the preceding claims, for a use in view of the treatment and/or prevention of pancreas cancer.
